# EUROPEAN PATENT SPECIFICATION

(11) **EP 2 280 929 B1**
(45) Date of publication and mention of the grant of the patent: **31.01.2018**
(21) Application number: 09757804.1
(22) Date of filing: 02.06.2009
(51) Int. Cl.: C07C 209/08, C07C 209/16, C07C 211/42, C07C 213/08, C07C 217/20, C07C 211/27

(54) **AN IMPROVED PROCESS FOR THE PREPARATION OF RASAGILINE**
VERBESSERTES VERFAHREN ZUR HERSTELLUNG VON RASAGILIN
METHODE AMELIOREE DE PREPARATION DE RASAGILINE

(30) Priority: 02.06.2008 IN KA09722008
(43) Date of publication of application: 09.02.2011
(73) Proprietor: Generics [UK] Limited, Potters Bar Hertfordshire EN6 1AG (GB)
(72) Inventor: GORE, Vinayak, Maharashtra 410208 (IN); MANOJKUMAR, Bindu, Maharashtra 410208 (IN); SONAWANE, Sandeep, Maharashtra 410208 (IN); KOKANE, Dattatrey, Maharashtra 410208 (IN); TANK, Sinderpal, Maharashtra 410208 (IN)
(74) Representative: Cooke, Richard Spencer
(86) International application number: PCT/GB2009/050610
(87) International publication number: WO 2009/147432

(56) References cited:
- US-A- 5 532 415
- HYDE ROBYN M. ET AL: NUCLEOSIDES & NUCLEOTIDES, vol. 21, no. 1, 2002, pages 45-54, XP009123681
- PRIEGO EVA-MARIA: SYNTHESIS, no. 3, 2001, pages 478-482, XP009123653

## Description

### Field of the invention

The present invention relates to an improved process for the preparation of rasagiline (1) and its pharmaceutically acceptable salts. In particular it relates to a process for preparing rasagiline (1) and its salts substantially free from impurities.

### Background of the invention

Rasagiline, represented by structural formula (1) and chemically named 1-(R)-(2-propynylamino)indan, is a selective and potent irreversible monoamine oxidase type B (MAO-B) inhibitor. It is currently marketed, as the mesylate salt, for the treatment of Parkinson's Disease (PD), both as monotherapy and as adjunct therapy to levodopa. Rasagiline (1) may also be useful for the treatment of dementia, Alzheimer's Disease, depression, hyperactive syndrome, stroke, brain ischemia, neurotrauma, schizophrenia and multiple sclerosis.

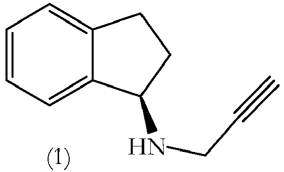

Rasagiline (1) was first described in US 5457133, in which the method of its preparation is a single step process comprising reacting (R)-1-aminoindan with propargyl chloride. The reaction is carried out in acetonitrile with potassium carbonate at a temperature of 60°C for 16 hours. The crude rasagiline (1) obtained by this process is very impure and has to be purified by column chromatography. The overall yield of rasagiline (1) from (R)-1-aminoindan is less than 44% and the process has other disadvantages such as a long reaction time and the need to isolate the product by column chromatography. In particular, the isolation of rasagiline (1) by chromatography makes the process much less amenable for scale-up for commercial production. In addition the reported process has the disadvantage of requiring the use of the more expensive component, (R)-1-aminoindan, in excess compared to the less expensive component, propargyl chloride.

Another process for the preparation of rasagiline (1), disclosed in patent application WO 95/11016, is very similar to the process disclosed in US 5457133, with the exception that propargyl benzenesulfonate is used instead of propargyl chloride. One of the examples disclosed in WO 95/11016 describes a process, which involves reaction of racemic 1-aminoindan with propargyl benzenesulfonate in the presence of 15% aqueous NaOH in toluene to form racemic rasagiline, which is then resolved to form rasagiline (1) by a crystallisation technique to isolate the required isomer. However, the reported process is low yielding and affords an impure product.

Another process for the preparation of rasagiline (1), disclosed in patent application WO 02/068376, involves the preparation of *N*-benzyl-1-aminoindan from indanone or 1-chloroindan and benzyl amine. Resolution of *N*-benzyl-1-aminoindan using L-tartaric acid and catalytic hydrogenolysis to remove the benzyl group affords (R)-1-aminoindan, which is further converted to rasagiline (1). This process suffers from the drawback of having many steps including protection and deprotection, and the overall yield for the preparation of (R)-1-aminoindan was reported to be a very low 19%.

Similar multi-step procedures have been reported in Organic Letters, vol. 8, no. 22, pages 5013-5016, 2006, US 5994408 and US 2006/0199974, but the reported processes suffer from the disadvantages of using expensive reagents; being multi-step, long processes; being low yielding; and/or producing impure products which require substantial purification.

As the commercial production of rasagiline (1) and rasagiline mesylate are of great importance and in view of the above disadvantages associated with the prior art, there is a real need for alternative and improved processes for the preparation of rasagiline (1) and rasagiline mesylate which do not involve multiple steps and further eliminate the need for cumbersome purification techniques. The alternative processes must be economical and high yielding and provide rasagiline (1) and rasagiline mesylate with a high degree of chemical and optical purity.

### Summary of the invention

The difficulties encountered in the prior art when preparing rasagiline (1) have been successfully overcome in the present invention.

The term "1-aminoindan" as used herein throughout the description and claims means 1-aminoindan and/or any salt, solvate or polymorphic form thereof, unless otherwise specified. The term "rasagiline" as used herein throughout the description and claims means rasagiline and/or any salt, solvate or polymorphic form thereof, unless otherwise specified. The current invention is particularly useful for the preparation of rasagiline free base and rasagiline mesylate.

Salts of 1-aminoindan and rasagiline include acid addition salts with suitable acids, including but not limited to inorganic acids such as hydrohalogenic acids (for example, hydrofluoric, hydrochloric, hydrobromic or hydroiodic acid) or other inorganic acids (for example, nitric, perchloric, sulfuric or phosphoric acid); or organic acids such as organic carboxylic acids (for example, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic or pamoic acid), organic sulfonic acids (for example, methanesulfonic, trifluoromethanesulfonic, ethanesulfonic, 2-hydroxyethanesulfonic, benzenesulfonic, toluene-p-sulfonic, naphthalene-2-sulfonic or camphorsulfonic acid) or amino acids (for example, ornithinic, glutamic or aspartic acid). A preferred salt of rasagiline is the methanesulfonic acid addition salt, also called rasagiline mesylate.

For the purposes of the present invention, 1-aminoindan, rasagiline or rasagiline mesylate is "racemic", if it comprises its enantiomers in a ratio of 60:40 to 40:60, for example as measured by chiral HPLC.

For the purposes of the present invention, 1-aminoindan, rasagiline or rasagiline mesylate is "enantiomerically enriched", if it comprises 60% or more of only one enantiomer, preferably 70% or more, preferably 80% or more, preferably 90% or more, for example as measured by chiral HPLC.

For the purposes of the present invention, 1-aminoindan, rasagiline or rasagiline mesylate is "enantiomerically pure", if it comprises 95% or more of only one enantiomer, preferably 98% or more, preferably 99% or more, preferably 99.5% or more, preferably 99.9% or more, for example as measured by chiral HPLC.
For the purposes of the present invention, 1-aminoindan, rasagiline or rasagiline mesylate is "substantially free" of chemical impurities, if it comprises less than 3% impurity, preferably less than 2%, preferably less than 1%, preferably less than 0.5%, preferably less than 0.1%, for example as measured by HPLC.
The present invention provides an efficient and economical synthesis of rasagiline (1) starting from 1-aminoindan, preferably from (R)-1-aminoindan, which is high yielding and affords the product with very high chemical and optical purity on a commercial scale, without the need for cumbersome purification techniques.
Accordingly, a first aspect according to the present invention provides a process for the preparation of rasagiline (1), comprising the step of:
(a) reacting 1-aminoindan with a propargyl compound H-C≡C-CH₂-X, or a protected form thereof, in the presence of an organic base selected from the group consisting of triethylamine, diisopropylethylamine, trimethylamine, diisopropylamine, pyridine, 4-dimethylaminopyridine (DMAP), N-methylmorpholine, 1,4- diazabicyclo- [2.2.2] octane (Dabco),1,5-diazabicyclo-[4.3.0]non-5-ene (DBN) and 1,8-diazabicyclo-[5.4.0]undec-7-ene (DBU), wherein X is a leaving group.
In a preferred embodiment of the process of the present invention, the 1-aminoindan is enantiomerically pure or enantiomerically enriched (R)-1-aminoindan, and the process further comprises the step of:
(b) isolating rasagiline (1) from the mixture obtained in step (a).
Preferably the rasagiline (1) isolated in step (b) is enantiomerically enriched, and most preferably the rasagiline (1) isolated in step (b) is enantiomerically pure.
In an alternative embodiment of the process of the present invention, the 1-aminoindan is racemic 1-aminoindan, and the process further comprises the steps of:
(b1) isolating racemic rasagiline from the mixture obtained in step (a), and
(b2) resolving the racemic rasagiline obtained in step (b1) to obtain rasagiline (1).

Preferably the rasagiline (1) obtained in step (b2) is enantiomerically enriched, and most preferably the rasagiline (1) obtained in step (b2) is enantiomerically pure.
Preferably, the process according to the first aspect of the present invention comprises the use of enantiomerically pure or enantiomerically enriched (R)-1-aminoindan. However, racemic 1-aminoindan can be used and the resulting racemic rasagiline can be converted to rasagiline (1) by following well-established and reported routes of resolution, for example, by following the procedure outlined in WO 95/11016.
Preferably, step (a) in the process according to the first aspect of the present invention is carried out in an organic solvent, wherein the organic solvent is preferably selected from the group comprising dimethyl sulfoxide, acetone, ethyl methyl ketone, tetrahydrofuran, dimethylformamide, dimethylacetamide, acetonitrile and mixtures thereof. More preferably, the organic solvent is dimethyl sulfoxide, acetone, ethyl methyl ketone, tetrahydrofuran, dimethylformamide, dimethylacetamide or a mixture thereof. Most preferably, the organic solvent is tetrahydrofuran.

Preferably, the organic base is Dabco, DBN or DBU. Most preferably, the organic base is DBU.

Preferably, the leaving group is a halo group, a carboxylic ester group or a sulfonate ester group. More preferably, the leaving group is a sulfonate ester group, more preferably a tosylate, besylate (benzene sulfonate), brosylate, nosylate, mesylate, tresylate, nonaflate or triflate group, more preferably a tosylate or besylate group, most preferably a tosylate group.
Preferably, in step (a) in the process according to the first aspect of the present invention, the 1-aminoindan is dissolved in the organic solvent at 0-5°C.

Preferably, the reaction temperature in step (a) in the process according to the first aspect of the present invention is between 10-40°C. More preferably, the reaction temperature is between 15-20°C.

Preferably, the rasagiline (1) formed in the process according to the first aspect of the present invention is further converted to a pharmaceutically acceptable salt, most preferably the mesylate salt. Pharmaceutically acceptable salts of rasagiline (1) include acid addition salts with suitable acids, including but not limited to inorganic acids such as hydrohalogenic acids (for example, hydrofluoric, hydrochloric, hydrobromic or hydroiodic acid) or other inorganic acids (for example, nitric, perchloric, sulfuric or phosphoric acid); or organic acids such as organic carboxylic acids (for example, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic or pamoic acid), organic sulfonic acids (for example, methanesulfonic, trifluoromethanesulfonic, ethanesulfonic, 2-hydroxyethanesulfonic, benzenesulfonic, toluene-p-sulfonic, naphthalene-2-sulfonic or camphorsulfonic acid) or amino acids (for example, ornithinic, glutamic or aspartic acid). A preferred salt of rasagiline (1) is the methanesulfonic acid addition salt, also called rasagiline mesylate.

Preferably, the rasagiline (1) or rasagiline salt are obtained in a yield of 70% or more, preferably 80% or more, preferably 90% or more, preferably 95% or more.

Preferably, the rasagiline (1) or rasagiline salt are obtained on a commercial scale, preferably in batches of 1kg or more, 10kg or more, 100kg or more, 500kg or more, or 1000kg or more.

Preferably, the rasagiline (1) or rasagiline salt obtained are enantiomerically pure, i.e. preferably they comprise 95% or more of only one enantiomer, preferably 98% or more, preferably 99% or more, preferably 99.5% or more, preferably 99.9% or more, for example as measured by chiral HPLC.

Preferably, the rasagiline (1) or rasagiline salt obtained are substantially free of chemical impurities, i.e. preferably they comprise less than 3% impurity, preferably less than 2%, preferably less than 1%, preferably less than 0.5%, preferably less than 0.1%, for example as measured by HPLC.

Preferably, the rasagiline (1) or rasagiline salt obtained comprise less than 0.8% of the impurity dipropargyl indanamine, preferably less than 0.5%, preferably less than 0.3%, preferably less than 0.1%, for example as measured by HPLC.

Preferably this optical and chemical purity is achieved without the use of chromatography.

A second aspect of the present invention provides rasagiline (1) or a pharmaceutically acceptable salt thereof, such as rasagiline mesylate, when prepared by a process according to the first aspect of the present invention.

A third aspect of the present invention provides enantiomerically pure rasagiline (1) or an enantiomerically pure pharmaceutically acceptable salt of rasagiline (1), such as enantiomerically pure rasagiline mesylate, when prepared by a process according to the first aspect of the present invention.

A fourth aspect of the present invention provides enantiomerically pure rasagiline (1) or an enantiomerically pure pharmaceutically acceptable salt of rasagiline (1), such as enantiomerically pure rasagiline mesylate.

A fifth aspect of the present invention provides rasagiline (1) substantially free of chemical impurities or a pharmaceutically acceptable salt of rasagiline (1), such as rasagiline mesylate, substantially free of chemical impurities.

The pharmaceutically acceptable salts of rasagiline (1) according to the second, third, fourth or fifth aspect of the present invention include acid addition salts with suitable acids, including but not limited to inorganic acids such as hydrohalogenic acids (for example, hydrofluoric, hydrochloric, hydrobromic or hydroiodic acid) or other inorganic acids (for example, nitric, perchloric, sulfuric or phosphoric acid); or organic acids such as organic carboxylic acids (for example, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic or pamoic acid), organic sulfonic acids (for example, methanesulfonic, trifluoromethanesulfonic, ethanesulfonic, 2-hydroxyethanesulfonic, benzenesulfonic, toluene-p-sulfonic, naphthalene-2-sulfonic or camphorsulfonic acid) or amino acids (for example, ornithinic, glutamic or aspartic acid). A preferred pharmaceutically acceptable salt of rasagiline (1) is the methanesulfonic acid addition salt, also called rasagiline mesylate. The rasagiline (1) or the pharmaceutically acceptable salt thereof, such as rasagiline mesylate, according to the second, third, fourth or fifth aspect of the present invention is suitable for treating or preventing Parkinson's Disease (PD) (including as monotherapy or as adjunct therapy to levodopa), dementia, Alzheimer's Disease, depression, hyperactive syndrome, stroke, brain ischemia, neurotrauma, schizophrenia or multiple sclerosis.

A sixth aspect of the present invention describes a pharmaceutical composition comprising the rasagiline (1) or the pharmaceutically acceptable salt thereof, such as rasagiline mesylate, according to the second, third, fourth or fifth aspect of the present invention. Preferably the pharmaceutical composition is suitable for treating or preventing Parkinson's Disease (PD) (including as monotherapy or as adjunct therapy to levodopa), dementia, Alzheimer's Disease, depression, hyperactive syndrome, stroke, brain ischemia, neurotrauma, schizophrenia or multiple sclerosis. Also described but not included in the present invention is a process for the preparation of a rasagiline analogue, comprising the step of:
(a) reacting a precursor amine with a propargyl compound H-C≡C-CH₂-X, or a protected form thereof, in the presence of an organic base, wherein X is a leaving group.

Rasagiline analogues include, but are not limited to selegiline, pargyline or clorgiline. The precursor amines for selegiline, pargyline and clorgiline are amines (I), (II) and (III) respectively: 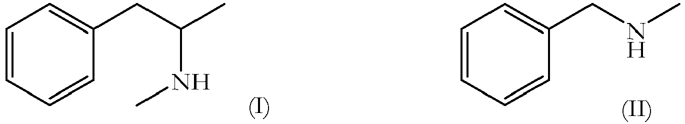 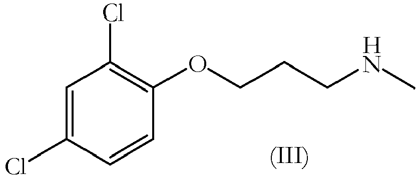 Preferably, enantiomerically pure or enantiomerically enriched precursor amine R-(I) is used in the process of the eighth aspect of the present invention for the preparation of selegiline:

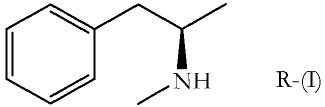

### Detailed description of the invention

The present invention provides a simple, convenient and inexpensive method for the preparation of rasagiline (1) and its pharmaceutically acceptable salts, such as rasagiline mesylate. The products obtained from the process of the present invention are surprisingly very pure without the need for cumbersome purification techniques. In particular, the present invention provides rasagiline (1) and its pharmaceutically acceptable salts, such as rasagiline mesylate, substantially free of the impurity dipropargyl indanamine. The dipropargyl indanamine is present at less than 0.8%, preferably less than 0.5%, more preferably less than 0.3%, and most preferably less than 0.1%.
The advantages of the present invention are the use of inexpensive, non-hazardous synthetic agents, simple and convenient process conditions, and a very fast synthetic process which has a strict control on the impurity profile of the resultant products which are of very high chemical and optical purity.
A preferred process of the present invention is the preparation of rasagiline (1), comprising the steps of reacting (R)-1-aminoindan with an organic base selected from the group consisting of triethylamine, diisopropylethylamine, trimethylamine, diisopropylamine, pyridine, 4-dimethylaminopyridine (DMAP), JV-methylmorpholine, 1,4- diazabicyclo- [2.2.2] octane (Dabco), 1,5-diazabicyclo-[4.3.0]non-5-ene (DBN) and 1,8- diazabicyclo-[5.4.0]undec-7-ene (DBU) in an organic solvent, then adding propargyl tosylate (also known as propargyl para-toluenesulfonate) to the mixture and stirring at 15-20°C. Surprisingly the choice of an organic base as described above as the reagent allows rasagiline (1) to be isolated from the reaction mixture in very high yield and purity. In addition, the choice of a propargyl sulfonate ester and an organic base appears to have a surprising synergistic effect in controlling the formation of impurities and increasing the rate of reaction.

Preferably, (R)-1-aminoindan is dissolved in an organic solvent, which preferably is selected from the group comprising dimethyl sulfoxide, dimethylformamide, dimethylacetamide, tetrahydrofuran, acetone, ethyl methyl ketone, acetonitrile and mixtures thereof. More preferably, the organic solvent is dimethyl sulfoxide, dimethylformamide, dimethylacetamide, tetrahydrofuran, acetone, ethyl methyl ketone or a mixture thereof. Most preferably, the organic solvent is tetrahydrofuran.

Preferably, the organic base is selected from the group comprising DBU, DBN and Dabco. In a most preferred embodiment, the organic base is DBU.
Preferably, the (R)-1-aminoindan and the organic base are dissolved in the organic solvent at 0-10°C, most preferably at about 0-5°C, and preferably stirred for about 30 minutes.
Preferably, propargyl tosylate is added slowly to the mixture of (R)-1-aminoindan and organic base at a temperature between about 0-10°C, the most preferred is a temperature in the range of between 0-5°C. Preferably, the reaction mixture is then kept between 10-40°C, preferably between 10-30°C, most preferably at about 15-20°C, preferably for about 4 hours.
A preferred embodiment of the process of the present invention is illustrated in Scheme 1. 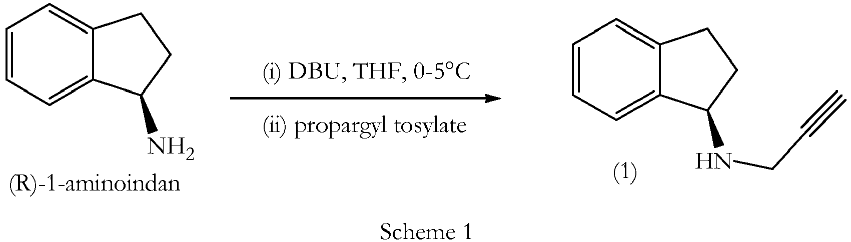 The reagents and solvents illustrated in Scheme 1 are merely illustrative of the present invention and the reactions are not limited by these reagents and solvents. Any suitable alternatives can be used as outlined above.

Preferably, the product (1) is obtained in a yield of 70% or more, preferably 80% or more, preferably 90% or more, preferably 95% or more.
The conversion of rasagiline (1) to rasagiline mesylate can be achieved by following well-established and reported routes of salt formation.
Preferably, the rasagiline (1) and rasagiline mesylate are obtained substantially free of chemical impurities.
Preferably, the rasagiline (1) and rasagiline mesylate are obtained on a commercial scale, preferably in batches of 1kg or more, 10kg or more, 100kg or more, 500kg or more, or 1000kg or more.

The pharmaceutical composition described but not included in the present invention can be a solution or suspension form, but is preferably a solid oral dosage form. Preferred dosage forms in accordance with the invention include tablets, capsules and the like which, optionally, may be coated if desired. Tablets can be prepared by conventional techniques, including direct compression, wet granulation and dry granulation. Capsules are generally formed from a gelatine material and can include a conventionally prepared granulate of excipients in accordance with the invention.

The pharmaceutical composition described but not included in the present invention typically comprises one or more conventional pharmaceutically acceptable excipient(s) selected from the group comprising a filler, a binder, a disintegrant, a lubricant, and optionally further comprises at least one excipient selected from colouring agents, adsorbents, surfactants, film-formers and plasticizers.
As described above, the pharmaceutical composition of the invention typically comprises one or more fillers such as microcrystalline cellulose, lactose, sugars, starches, modified starches, mannitol, sorbitol and other polyols, dextrin, dextran or maltodextrin; one or more binders such as lactose, starches, modified starch, maize starch, dextrin, dextran, maltodextrin, microcrystalline cellulose, sugars, polyethylene glycols, hydroxypropyl cellulose, hydroxypropyl methyl cellulose, ethyl cellulose, hydroxyethyl cellulose, methyl cellulose, carboxymethyl cellulose, gelatine, acacia gum, tragacanth, polyvinylpyrrolidone or crospovidone; one or more disintegrating agents such as croscarmellose sodium, cross-linked polyvinylpyrrolidone, crospovidone, cross-linked carboxymethyl starch, starches, microcrystalline cellulose or polyacrylin potassium; one or more different glidants or lubricants such as magnesium stearate, calcium stearate, zinc stearate, calcium behenate, sodium stearyl fumarate, talc, magnesium trisilicate, stearic acid, palmitic acid, carnauba wax or silicon dioxide.

If required, the pharmaceutical composition may also include surfactants and other conventional excipients.

If the solid pharmaceutical formulation is in the form of coated tablets, the coating may be prepared from at least one film-former such as hydroxypropyl methyl cellulose, hydroxypropyl cellulose or methacrylate polymers which optionally may contain at least one plasticizer such as polyethylene glycols, dibutyl sebacate, triethyl citrate, and other pharmaceutical auxiliary substances conventional for film coatings, such as pigments, fillers and others.
Experimental details of a preferred example of the invention are given below.

### Example

### Rasagiline (1)

(R)-1-Aminoindan (1 equivalent) was dissolved in dry THF (3 vol) and cooled to 0-5°C. DBU (1.5 equivalents) was added slowly and the resultant mixture stirred for 30 minutes before propargyl tosylate (1.25 equivalents) was added dropwise. The reaction mixture was stirred for 4 hours at 15-20°C. After completion of the reaction, the THF was removed under vacuum and water (3 vol) added. The product was extracted with DCM (3 x 3 vol), followed by washing with 10% aqueous NaOH (2 x 3 vol) and water (2 x 3 vol). The DCM was removed under vacuum at 40°C to obtain the product as a light yellow oil.
Molar yield = 80-82%
Chemical purity = 64.33% (measured by HPLC)

### Rasagiline Mesylate

Rasagiline (1) (1 equivalent) was dissolved in IPA (3 vol) and cooled to 0-5°C before a solution of methanesulfonic acid (1 equivalent) in IPA (2 vol) was added. The mixture was stirred for 30 minutes, filtered and the resultant solid washed with IPA (2 vol) and then with TBME (2 vol). The solid was dried under vacuum at 10mbar at 40°C to obtain the product as a white solid.
Molar yield = 47%
Chemical purity = 99.84% (measured by HPLC)
Optical purity = 100% (measured by chiral HPLC) (no (S)-enantiomer detected)

The difficulties encountered in the prior art when preparing rasagiline (1) have been successfully overcome by the process of the present invention.

## Claims

1. A process for the preparation of rasagiline (1), comprising the step of:
(a) reacting 1-aminoindan with a propargyl compound H-C≡C-CH₂-X, or a protected form thereof, in the presence of an organic base selected from the group consisting of triethylamine, diisopropylethylatnine, trimethylamine, diisopropylamine, pyridine, 4-dimethylaminopyridine (DMAP), *N*-methylmorpholine, 1,4-diazabicyclo-[2.2.2]octane (Dabco), 1,5-diazabicyclo-[4.3.0]non-5-ene (DBN) and 1,8-diazabicyclo-[5.4.0]undec-7-ene (DBU), wherein X is a leaving group.

2. A process according to claim 1, wherein the 1-aminoindan is enantiomerically pure or enantiomerically enriched (R)-1-aminoindan, and wherein the process further comprises the step of:
(b) isolating rasagiline (1) from the mixture obtained in step (a).

3. A process according to claim 1, wherein the 1-aminoindan is racemic 1-aminoindan, and wherein the process further comprises the steps of:
(b1) isolating racemic rasagiline from the mixture obtained in step (a), and
(b2) resolving the racemic rasagiline obtained in step (b1) to obtain rasagiline (1).

4. A process according to any preceding claim, wherein step (a) is carried out in:
(i) an organic solvent; and/or
(ii) an organic solvent selected from the group comprising dimethyl sulfoxide, acetone, tetrabydrofuran, ethyl methyl ketone, dimethylformamide, dimethylacetamide, acetonitrile and mixtures thereof; and/or
(iii) tetrahydrofuran.

5. A process according to any preceding claim, wherein the organic base is DBU.

6. A process according to any preceding claim, wherein the leaving group is:
(i) a halo group, a carboxylic ester group or a sulfonate ester group; or
(ii) a tosylate, mesylate, brosylate, nosylate, besylate, tresylate, nonaflate or triflate; or
(iii) a tosylate or besylate.

7. A process according to claim 4, wherein the 1-aminoindan is dissolved in the organic solvent at 0-5°C.

8. A process according to any preceding claim, wherein the reaction temperature in step (a) is:
(i) between 10-40°C or
(ii) between 15-20°C.

9. A process according to any preceding claim, wherein the rasagiline (1) formed is converted to a pharmaceutically acceptable salt, such as the mesylate salt.

## Patentansprüche

1. Verfahren zur Herstellung von Rasagilin (1), umfassend den Schritt des:
(a) Umsetzens von 1-Aminoindan mit einer Propargylverbindung H-C≡C-CH₂-X oder einer geschützten Form davon in Gegenwart einer organischen Base, die ausgewählt ist aus der Gruppe bestehend aus Triethylamin, Diisopropylethylamin, Trimethylamin, Diisopropylamin, Pyridin, 4-Dimethylaminopyridin (DMAP), N-Methylmorpholin, 1,4-Diazabicyclo-[2.2.2]octan (Dabco), 1,5-Diazabicyclo-[4.3.0]non-5-en (DBN) und 1,8-Diazabicyclo-[5.4.0]undec-7-en (DBU), wobei X eine Abgangsgruppe ist.

2. Verfahren nach Anspruch 1, wobei das 1-Aminoindan enantiomer reines oder enantiomer angereichertes (R)-1-Aminoindan ist und wobei das Verfahren weiterhin den Schritt des:
(b) Isolierens von Rasagilin (1) aus der in Schritt (a) erhaltenen Mischung
umfasst.

3. Verfahren nach Anspruch 1, wobei das 1-Aminoindan racemisches 1-Aminoindan ist und wobei das Verfahren weiterhin die Schritte des:
(b1) Isolierens von racemischem Rasagilin aus der in Schritt (a) erhaltenen Mischung und
(b2) Spaltens des in Schritt (b1) erhaltenen racemischen Rasagilins unter Erhalt von Rasagilin (1)
umfasst.

4. Verfahren nach einem der vorhergehenden Ansprüche, wobei Schritt (a) in:
(i) einem organischen Lösungsmittel und/oder
(ii) einem organischen Lösungsmittel, das ausgewählt ist aus der Gruppe umfassend Dimethylsulfoxid, Aceton, Tetrahydrofuran, Ethylmethylketon, Dimethylformamid, Dimethylacetamid, Acetonitril und Mischungen davon und/oder
(iii) Tetrahydrofuran
durchgeführt wird.

5. Verfahren nach einem der vorhergehenden Ansprüche, wobei es sich bei der organischen Base um DBU handelt.

6. Verfahren nach einem der vorhergehenden Ansprüche, wobei es sich bei der Abgangsgruppe um:
(i) eine Halogengruppe, eine Carbonsäureestergruppe oder eine Sulfonatestergruppe oder
(ii) ein Tosylat, Besilat, Brosylat, Nosylat, Mesylat, Tresylat, Nonaflat oder Triflat oder
(iii) ein Tosylat oder Besilat
handelt.

7. Verfahren nach Anspruch 4, wobei das 1-Aminoindan bei 0-5 °C im organischen Lösungsmittel gelöst wird.

8. Verfahren nach einem der vorhergehenden Ansprüche, wobei die Reaktionstemperatur in Schritt (a):
(i) zwischen 10-40 °C oder
(ii) zwischen 15-20 °C
liegt.

9. Verfahren nach einem der vorhergehenden Ansprüche, wobei das gebildete Rasagilin (1) in ein pharmazeutisch annehmbares Salz wie das Mesylatsalz umgewandelt wird.

## Revendications

1. Procédé de préparation de rasagiline (1), comprenant l'étape de :
(a) réaction de 1-aminoindane avec un composé de propargyle H-C≡C-CH₂-X, ou une de ses formes protégées, en présence d'une base organique choisie dans le groupe constitué de la triéthylamine, de la diisopropyléthylamine, de la triméthylamine, de la diisopropylamine, de la pyridine, de la 4- diméthylaminopyridine (DMAP), de la N-méthylmorpholine, du 1,4-diazabicyclo-[2.2.2]octane (Dabco), du 1,5-diazabicyclo-[4.3.0]non-5-ène (DBN) et du 1,8-diazabicyclo-[5.4.0]undéc-7-ène (DBU), où X est un groupe partant.

2. Procédé selon la revendication 1, dans lequel le 1-aminoindane est un énantiomère pur ou un énantiomère enrichi (R)-1-aminoindane, et où le procédé comprend en outre l'étape :
(b) d'isolement de la rasagiline (1) à partir du mélange obtenu dans l'étape (a) .

3. Procédé selon la revendication 1, dans lequel le 1-aminoindane est un 1-aminoindane racémique, et où le procédé comprend en outre les étampes :
(b1) d'isolement de la rasagiline racémique du mélange obtenu dans l'étape (a), et
(b2) de résolution de la rasagiline racémique obtenue dans l'étape (b1) afin d'obtenir la rasagiline (1).

4. Procédé selon une quelconque revendication précédente, dans lequel l'étape (a) est effectuée dans :
(i) un solvant organique ; et/ou
(ii) un solvant organique choisi dans le groupe comprenant le diméthyl sulfoxyde, l'acétone, le tétrahydrofurane, l'éthyl méthyl cétone, le diméthylformamide, le diméthylacétamide, l'acétonitrile et de mélanges de ceux-ci ; et/ou
(iii) le tétrahydrofurane.

5. Procédé selon une quelconque revendication précédente, dans lequel la base organique est le DBU.

6. Procédé selon une quelconque revendication précédente, dans lequel le groupe partant est :
(i) un groupe halogéné, un groupe ester carboxylique ou un groupe ester sulfonate ; ou
(ii) un tosylate, un bésylate, un brosylate, un nosylate, un mésylate, un trésylate, un nonaflate ou un triflate ; ou
(iii) un tosylate ou un bésylate.

7. Procédé selon la revendication 4, dans lequel le 1-aminoindane est dissout dans le solvant organique à 0 à 5 °C.

8. Procédé selon une quelconque revendication précédente, dans lequel la température de réaction dans l'étape (a) est :
(i) entre 10 et 40 °C ; ou
(ii) entre 15 et 20 °C.

9. Procédé selon une quelconque revendication précédente, dans lequel la rasagiline (1) formée est convertie en un sel pharmaceutiquement acceptable, tel qu'un sel mésylate.
